**Europäisches Patentamt**

**European Patent Office**

**Office européen des brevets**

(11) Publication number: **0 187 594**
**B1**

(12) **EUROPEAN PATENT SPECIFICATION**

(45) Date of publication of patent specification: **21.03.90**

(21) Application number: **85402539.2**

(22) Date of filing: **18.12.85**

(51) Int. Cl.⁵: **B 01 J 29/30, C 10 G 3/00,
C 07 C 1/20, B 01 J 29/36**

(54) Zeolite catalysts.

(30) Priority: **03.01.85 CA 471463**

(43) Date of publication of application:
**16.07.86 Bulletin 86/29**

(45) Publication of the grant of the patent:
**21.03.90 Bulletin 90/12**

(84) Designated Contracting States:
**DE FR IT NL**

(56) References cited:
**EP-A-0 124 998
DE-A-1 442 901
DE-A-3 228 270**

**CHEMICAL ABSTRACTS, vol. 97, no. 26, 27th
December 1982, abstract no. 219327y, page 193,
Columbus, Ohio, US; K. MUELLER et al.:
"Fischer-Trophesis on polyfunctional
manganese/iron-pentasil zeolite catalysts", &
STUD. SURF. SCI. CATAL. 1982, 12(Met.
Microstruct. Zeolites: Prep. - Prop. - Appl.),
267-74**

(73) Proprietor: **THE ASBESTOS INSTITUTE
1130 Sherbrooke Street West
Montreal Quebec H3A 2M8 (CA)**

(72) Inventor: **Le Van Mao, Raymond
3832 de la Peltrie Street
Montreal Quebec H3S 1V3 (CA)**

(74) Representative: **Moncheny, Michel et al
c/o Cabinet Lavoix 2 Place d'Estienne d'Orves
F-75441 Paris Cedex 09 (FR)**

Courier Press, Leamington Spa, England.

## EP 0 187 594 B1

**Description**

Prior Art — Background of the Invention

Because of large availability of methanol in the industrial world, it has for many years been considered as the most desirable material for obtaining light olefins, aromatic and non-aromatic liquids in the gasoline boiling range. Methanol is also the intermediate of the process starting from synthesis gas. This synthesis gas (a mixture of carbon monoxide and hydrogen) can be derived from natural gases, biomass, coal or heavy oils.

Since light olefins and liquid hydrocarbons (in particular, aromatics) are the most commercially valuable products in the catalytic conversion of methanol and dimethyl ether (dehydration derivative of methanol), high yields in such hydrocarbons are desired in the industrial viewpoint.

Many attempts have been done to find the ideal catalyst. Crystalline aluminosilicate zeolites are the most indicated for this purpose, due mainly to their ordered and molecular-sized framework structure. Among them, the ZSM—5 type zeolites show high performances both in terms of methanol conversion and product selectivities. However, under normal reaction conditions, light paraffin production occurs for more than the third of the hydrocarbon yield as can be seen from Table 1.

EP 0 187 594 B1

TABLE 1: The prior art

| Catalyst | Reaction Conditions | | Methanol conversion into hydrocarbons (%) (1) | Product selectivities (%) | | | | Ole+Liq (liq= Ar+C5+) | Ole + Ar | Ole/Ar |
|---|---|---|---|---|---|---|---|---|---|---|
| | T (°C) | Press. (atm) | | $C_1$-$C_4$ Paraffins | $C_2$-$C_4$ olefins (Ole) | Aromatics (Ar) | Non-Aromatics ($C_5^+$) | | | |
| ZSM-5 zeolite (A) | 427 | 1 | 99 | 42 | 10 | 41 | 7 | 58 | 51 | 0.3 |
| | 427 | 0.25 | 99 | 22 | 38 | 20 | 20 | 78 | 58 | 1.8 (2) |
| | 427 | 0.07 | 99 | 15 | 78 | 2 | 5 | 85 | 80 | 39.0 (2) |
| ZSM-5 Zeolite (B) | 371 | 1 | 99 | 35(3) | 3(3) | 41 | 21 | 65 | 44- | 0.1 |
| ZSM-5 Zeolite promoted with trimethylphosphite (C) | 385 | 1 | 31 | 5 | 39 | 20 | 36 | 95 | 60 | 1.9 |
| | 465 | 1 | 88 | 4 | 62 | 8 | 26 | 96 | 70 | 7.8 |
| | 560 | 1 | 99 | 5 | 66 | 12 | 16 | 94 | 78 | 5.5 |
| 13X Zeolite bearing Mg and Mn (D) | 400 | 1 | 91 | 19 | 81 | – | – | 81 | 81 | ∞(4) |
| ZSM-5 Zeolite with incorporation of one metal of group $I_b$,$II_a$, $II_b$,$III_a$,$IV_a$ or VIII (E) | 400 | 1 | 99 | (NR) (31) | (NR) | 37 | 32 | NR | NR | NR (5) |

REMARKS: (1) Carbon atom basis  (2) Subatmospheric pressure of methanol  (3) reported in Chang C.D. et al J. Cat. 86, 289 (1984)

(4) no liquid hydrocarbons produced  (5) not reported but likely similar to the case of US3,894,103

PATENTS: A.  US 4,025,575 (Mobil Oil)  B. US 3,894,103 ($SiO_2$/$Al_2O_3$=100) (Mobil Oil)  C.  US 3,911,041 (Mobil Oil)

D.  Ger. Offen 2,755,229 (Hoeschst A.G.)  E. US 3,894,104 (example 6, Zn) (Mobil Oil).

EP 0 187 594 B1

Modifications of reaction parameters (for example, by using very low-subatmospheric partial pressure of methanol) can decrease the light paraffin production and increase the light olefin yield. However, not only the aromatic formation is also depressed (see Table 1), but also the methanol conversion per pass per unit weight of catalyst is very limited.

Modifications of the catalyst characteristics (for example $SiO_2/Al_2O_3$ ratio) leads to a high liquid hydrocarbon yield with relatively high aromatic content and a high production of light paraffins and a very low production of light olefins (see Table 1).

Modifications of the zeolite chemical composition by incorporating extraneous components lead to one of these two product distributions:

a) large production of light olefins with significant (or smaller) production of light paraffins, negligible (or much reduced) production of aromatic and non-aromatic liquid hydrocarbons. For this category, we can mention the ZSM—5 zeolite promoted with trimethylphosphite or modified with silica, and the 13XX zeolite bearing Mg and Mn (see Table 1). One of the drawbacks of these catalysts is their relatively low methanol conversion within the range of reaction temperature (370—450°C); or their very reduced yield in liquid hydrocarbons;

b) large production of liquid hydrocarbons (with a relatively high aromatic content), and significant production of light paraffins and a very reduced production of light olefins.

For this category, we can mention the case of the ZSM—5 zeolite into which is incorporated Zn or an ion of one among the following groups: $I_b$, $II_a$, $II_b$, $III_a$, $IV_a$ or VIII (see Table 1).

Objects of the Present Invention

An ideal catalytic system for the conversion of methanol into hydrocarbons do have all the following catalytic performance under normal reaction conditions as tested for the parent ZSM—5 zeolite (i.e. atmospheric pressure, temperature ranging from 370 to 450°C, WHSV ranging from 0.1 to 10 $hr^{-1}$):

1) high methanol conversion into hydrocarbons, close to 100% (carbon atom based calculation);
2) low gaseous paraffin production;
3) high cumulative production of light olefins and liquid hydrocarbons, i.e. equal to or more than 90%;
4) high cumulative production of light olefins and aromatics;
5) a) equilibrated production of light olefins and aromatics, i.e. Ole/Ar ratio close to 1; and
   b) possibility of shifting this Ole/Ar ratio to higher value, without changing the previous figure, i.e. keeping 1), 2), 3) and 4) constant.

Summary of the Invention

The present invention relates to novel metal loaded zeolite catalysts selected from pentasil zeolite ZSM—5 and pentasil zeolite-asbestos composite catalyst.

The pentasil zeolite ZSM—5 catalyst is characterized by having a portion of its internal acid reaction sites replaced with zinc ions and by having manganese ions absorbed on the external surface of the zeolite.

On the other hand, the pentasil zeolite-asbestos composite catalyst comprises a pentasil zeolite within a magnesium and iron leached asbestos micro-matrix and is characterized by having a small portion of the internal acid reaction sites of the pentasil zeolite replaced by zinc ions and by having manganese ions absorbed on the external surface thereof while the magnesium and iron leached asbestos micro-matrix is doped internally with zinc ions and externally with manganese ions. In each of the novel catalysts of the present invention the amount of zinc varies from 0.1 to 1.0% w/w of the metal content while the amount of manganese varies from 0.2 to 5% w/w of the metal content.

A definition of pentasil is to be found in an article entitled "Pentasil family of high silica crystalline materials" in Proc. Conf. London (April 1979, p. 133—139).

The novel concept of loading Zn and Mn onto ZSM—5 zeolite or onto composite ZSM—5 zeolite/ asbestos materials leads to the following catalytic performances at 400°C and 450°C under atmospheric pressure and at 2.4 $hr^{-1}$ as WHSV (see Table 2):

1) methanol conversion into hydrocarbons close to 100% or at least higher than 85% (carbon atom based calculation);
2) light paraffin selectivity lower than 10%;
3) cumulative selectivity of light olefins and liquid hydrocarbons, equal to or higher than 90%;
4) cumulative selectivity of light olefins and aromatics:
   a) close to 70% at 400°C;
   b) equal to or higher than 80% at 450°C;
5) a) Ole/Ar = 1.1 with moderate Mn loading;
   b) Ole/Ar higher than 1.1 with higher Mn loading, without changing items 1), 2), 3) and 4).

The catalytic performances are illustrated in Table 2.

4

TABLE 2: The present invention

| Catalyst | Zn (%) | Mn (%) | Reaction conditions | | Methanol conversion into hydrocarbons(2) | Product selectivities (%) | | | | Ole+ Liq. (Liq= Ar+C$_5^+$) | Ole+ Ar | Ole/ Ar |
|---|---|---|---|---|---|---|---|---|---|---|---|---|
| | | | T (°C) | pressure (atm) (1) | | C$_1$-C$_4$ Para- ffins | C$_2$-C$_4$ Olefins (Ole) | Aromatics (Ar) | Non- Aromatics (C$_5^+$) | | | |
| Zn-Mn doped ZSM-5 zeolite | 0.37 | 1.17 | 400 | 0.9 | 99 | 11 | 37 | 31 | 21 | 89 | 68 | 1.2 |
| | | | 450 | 0.9 | 93 | 6 | 46 | 33 | 15 | 94 | 79 | 1.4 |
| Zn-Mn doped composite ZSM5/ asbestos cat. (high MLD)(3) | 0.23 | 1.10 | 400 | 0.9 | 99 | 10 | 37 | 34 | 19 | 90 | 71 | 1.1 |
| | | | 450 | 0.9 | 98 | 7 | 43 | 39 | 11 | 93 | 82 | 1.1 |
| | 0.23 | 2.08 | 400 | 0.9 | 98 | 7 | 47 | 21 | 25 | 93 | 68 | 2.2 |
| | | | 450 | 0.9 | 97 | 5 | 50 | 32 | 13 | 95 | 82 | 1.6 |
| Zn-Mn doped composite ZSM5/ asbestos cat. (Med. MLD)(3) | 0.32 | 1.60 | 400 | 0.9 | 91 | 5 | 50 | 18 | 27 | 95 | 68 | 2.8 |
| | | | 450 | 0.9 | 86 | 6 | 51 | 25 | 18 | 94 | 76 | 2.0 |

(1) total pressure = 1 atm (balance with nitrogen)

(2) carbon atom basis (%)

(3) magnesium leaching degree. High MLD > 90%. Medium MLD = 80 ÷ 90%.

The present invention also relates to the conversion of methanol (or dimethyl ether) into hydrocarbons over Zn and Mn loaded catalysts of the zeolite type.

The invention also is concerned with the high cumulative production of light olefins and gasoline boiling range hydrocarbons having a high content in aromatics. It is also a feature of the present invention to provide a catalytic system capable of giving a distribution which can be changed from an equilibrated production of light olefins and aromatics to a light olefin richer production, the cumulative production of light olefins and aromatics being kept constant at a high value. To achieve this product distribution change, it is sufficient to monitor the Mn content in the final catalyst.

The zeolites which are directly concerned by the present invention belong to the family called ZSM or pentasil zeolite family, namely ZSM—5 type zeolites.

The basic zeolite system can be a pure ZSM—5 zeolite under acid form (H—ZSM—5) or a composite ZSM—5 zeolite/asbestos material. The latter composite material is prepared by a multi-step process which includes the partial leaching of the metallic components (magnesium and iron) from the chrysotile asbestos fibres, folowed by the "in situ" zeolite crystallization and ended by the incorporation of the acid sites into the zeolite lattice.

The Zn loading into the zeolite catalysts is performed under ion-exchange conditions. While H—ZSM—5 material requires very severe exchange conditions to obtain the desired content in Zn ions, the composite ZSM—5/asbestos material needs very mild conditions for incorporating Zn ions with the desired content. It is expected that with H—ZSM—5 zeolite, the $Zn^{2+}$ ions do replace some of the acid sites mainly located within the zeolite pores.

On the other hand, it is found that with the composite ZSM—5/asbestos material, the loading of Zn ions was accompanied by an important loss of Fe ions (the Mg content remained substantially constant): this means that a small part of Zn ions was exchanged with the acid sites of the zeolite component while a large part of these ions was incorporated into the remnants of the chrysotile asbestos.

The Mn loading was simply performed by dry impregnation of a solution of manganese salt onto the Zn-loaded catalyst.

To achieve the desired catalytic performance, in both cases the Zn metal content of the final catalysts must be less than 1% (wt/wt of catalyst), preferably between 0.1% (wt/wt) and 0.6% (wt/wt) and most preferably between 0.1 and 0.3%. The Mn metal content must be at least 0.2% (wt/wt), up to 5% (wt/wt) and preferably up to 2.5% (wt/wt).

The Ole/Ar ratio depends strongly on the Mn/Zn weight or atom ratio. For example, under reaction conditions as described in the section previously and with catalysts which exhibit a Mat/Al ratio of about 30/1, the following dependance can be exemplified for a Zn content of 0.2 to 0.4% wt/wt:

MN/Zn = 2 (weight basis) = 2.3 (atom basis), most of the present catalysts exhibit a Ole/Ar ratio close to 1.

Mn/Zn = 8 (weight basis) = 9.5 (atom basis), most of the present catalysts exhibit a Ole/Ar ratio close to 2.

Description of the Invention

The basic composite pure pentasil ZSM—5 zeolite and pentasil ZSM—5 zeolite-asbestos composite catalysts of the present invention are prepared according to the following procedure:

Composite ZSM—5 zeolite/asbestos material

The fibrous asbestos used in the examples of the present invention is of type 7TF—12 chrysotile asbestos, with short fibres, but the present procedure for preparing and testing the pentasil zeolite-asbestos composite catalysts is applicable to all other types of fibrous asbestos.

The starting material, 7TF—12 asbestos, has the following composition (% by weight):

| | | |
|---|---|---|
| $SiO_2$ | : | 42.8% |
| MgO | : | 50.2% |
| $Fe_2O_3$ | : | 6.6% (under FeO and $Fe_3O_4$ forms) |
| $Al_2O_3$ | : | 0.1% |
| $Na_2O$ | : | 0.1% |

The catalyst is prepared by partially leaching the magnesium (and the iron) out of the asbestos materials by acid attack.

In particular, aqueous solutions of HCl are used to perform the leaching operation. Table 3 reports the conditions of preparation and the composition of the leached asbestos (Alix).

| ALIX | Amount of 7TF-12 asbestos (g) | HCl solution | | Heating Time (80°C) | Product | | | | | | |
|------|------|------|------|------|------|------|------|------|------|------|------|
| | | N | ml | | (dehyd) g | $SiO_2$ (%) | $Al_2O_3$ (%) | $Na_2O$ (%) | MgO (%) | $Fe_2O_3$ (%) | MLD (%) |
| 001/2 | 100 | 3.0 | 1000 | 7.0 hr | 40 | 98.2 | 0.36 | 0.14 | 1.06 | 0.28 | 98 |
| 013 | 100 | 2.4 | 1000 | 4.5 hr | 44 | 89.0 | 0.51 | 0.14 | 4.14 | 6.22 | 92 |
| 025 | 200 | 3.0 | 2000 | 5.0 hr | 87 | 90.6 | 0.32 | 0.14 | 2.95 | 2.57 | 94 |
| 020/2 | 100 | 2.4 | 1000 | 4.0 hr | 42 | 91.8 | 0.38 | 0.14 | 3.38 | 4.35 | 93 |
| 024 | 120 | 2.4 | 1200 | 3.5 hr | 53 | 88.0 | 0.35 | 0.13 | 5.88 | 5.65 | 88 |
| 018/1 | 100 | 2.4 | 1000 | 3.5 hr | 45 | 85.2 | 0.36 | 0.16 | 6.60 | 7.65 | 87 |
| 018/2 | 100 | 2.4 | 1000 | 3.5 hr | 43 | 86.1 | 0.32 | 0.14 | 6.23 | 7.15 | 88 |
| 027 | 150 | 2.4 | 1500 | 2.5 hr | 71 | 82.6 | 0.43 | 0.19 | 9.39 | 7.68 | 81 |
| 030 | 150 | 2.4 | 1500 | 2.5 hr | 69 | 83.7 | 0.22 | 0.16 | 8.99 | 6.98 | 82 |
| 017 | 100 | 2.4 | 1000 | 2.5 hr | 41 | 81.8 | 0.45 | 0.12 | 10.10 | 7.59 | 80 |
| 016 | 100 | 2.4 | 1000 | 1.25 hr | 47 | 82.0 | 0.18 | 0.13 | 10.53 | 7.20 | 80 |

EP 0 187 594 B1

The leaching of magnesium via strong mineral acid treatment and under drastic conditions (high concentration acid, several hours heating) can solubilize most of the magnesium (and other metallic components) leaving behind a collapsed macro-structure of geliform silica. Mild acid treatment with diluted aqueous solutions of strong mineral acids or attack with weak acids like acetic acid, oxalic acid, etc., may preserve the chrysotile macro-structure, the magnesium content of which depends on the acid treatment conditions. Thus, by controlling the magnesium leaching operation conditions, it is possible to obtain a predetermined magnesium leaching degree.

The following example can be considered as typical procedure for the magnesium leaching step: 100 g of chrysotile asbestos fibres (7TF—12 grade) were suspended in a solution prepared from 200 ml of concentrated HCl and 800 ml of distilled water.

The suspension was heated at 70°—80°C with rapid stirring for 2.5 hours. Then, 2000 ml of distilled water were added and the resulting suspension was allowed to stand for 12 hours at room temperature. The suspension was filtered and washed with 2 litres of distilled water at room temperature. The solid was dried at 120°C for 12 hours and the chemical composition of the resulting material, called Alix 017, is reported in Table 3.

The magnesium leaching degree (MLD) of the Alix (Table 3) is defined as:

$$(MLD) = \frac{(MgO)_i - (MgO)_f}{(MgO)_i} \times 100 \ (\%)$$

where $(MgO)_i$ and $(MgO)_f$ are the magnesium contents (dried oxide basis) of the asbestos fibres and of the Alix, respectively.

ZSM—5 zeolite was crystallized using the Alix as starting material. To have the required conditions for preparing efficient catalysts for the conversion of methanol, sodium aluminate was added to the suspension which contains also NaOH and tetrapropylammonium ion (TPA) as synthesizing agent for the ZSM—5 zeolite.

The following example can be considered as typical procedure for the step of the ZSM—5 zeolite crystallization:

25.0 g of the Alix 017 were suspended in a solution prepared from 40.0 g of tetrapropylammonium bromide (TPA Br from Fisher Sc. Co., > 98%) and 2.5 g of NaOH (Fisher Sc. Co.) dissolved in 160 ml of distilled water. The suspension was heated at 70°—80° for 1 hour under vigorous stirring. Then, a solution of 1.5 g of sodium aluminate (Fisher Co., % weight composition: $Al_2O_3$ = 46.8%, $Na_2O$ = 28.4) in 20 ml of distilled water was added. Heating was continued at 70°—80°C with vigorous stirring for 10 minutes.

The suspension was transferred into a polypropylene bottle which was then put into a Sparr autoclave and heated at 170°C (± 5°C) for 10 days.

After cooling, the suspension was discharged and filtered; the solid was washed with distilled water until the washing liquid had a pH lower than 9 and then dried at 120°C for 12 hours and finally activated in the air at 550°C for 12 hours. The resulting material, called A—48, was a precursor for the composite catalyst HA—48.

Table 4 reports the conditions for synthesizing the ZSM—5 zeolite component in the composite catalyst precursors (A).

TABLE 4: Synthesis of ZSM-zeolite from silica gel and Alix

| Catalyst Precursor | Alix | W (g) | Silica gel (g) | Na aluminate (g) | TBA Bromide (g) | NaOH (g) | Added Water (ml) | Synthesis T (°C) | days | Product[3] (g) |
|---|---|---|---|---|---|---|---|---|---|---|
| P-38 | – | – | 44[1] | 1.5 | 40 | 2.5 | 160 | 170 | 10 | 22.4 |
| P-68 | – | – | 25[2] | 1.8 | 40 | 2.5 | 160 | 170 | 10 | 24.4 |
| A-39 | 001/2 | 24.0 | – | 1.5 | 40 | 2.5 | 180 | 170 | 10 | 20.0 |
| A-45 | 013 | 24.1 | – | 1.5 | 40 | 2.5 | 180 | 170 | 10 | 19.4 |
| A-62 | 025 | 35.0 | – | 2.0 | 40 | 2.5 | 180 | 170 | 10 | 30.3 |
| A-56 | 020/2 | 25.0 | – | 1.5 | 40 | 2.5 | 180 | 170 | 10 | 21.3 |
| A-58 | 024 | 45.0 | – | 2.7 | 40 | 2.5 | 180 | 170 | 10 | 46.5 |
| A-49 | 018/1 | 25.0 | – | 1.5 | 40 | 2.5 | 180 | 170 | 10 | 21.1 |
| A-54 | 018/2 | 30.0 | – | 1.8 | 40 | 2.5 | 180 | 170 | 10 | 25.3 |
| A-64 | 027 | 35.0 | – | 2.0 | 40 | 2.5 | 180 | 170 | 10 | 30.7 |
| A-69 | 030 | 66.0 | – | 4.2 | 80 | 5.0 | 360 | 170 | 10 | 56.7 |
| A-48 | 017 | 25.0 | – | 1.5 | 40 | 2.5 | 180 | 170 | 10 | 20.4 |
| A-60 | 016 | 35.0 | – | 2.0 | 40 | 2.5 | 180 | 170 | 10 | 31.8 |

Silica gel Baker:  (1) = 60% in $SiO_2$;  (2) = 90% in $SiO_2$

(3) The product is dried 12 h at 120°C and then a further 12 h at 550°C.

The composite ZSM—5 zeolite/asbestos precursors were submitted to ion-exchange with a $NH_4Cl$ aqueous solution.

The following example can be considered as typical procedure for the step of ion-exchanging with $NH_4$ ions.

20.0 g of sample A—48 were brought in contact with an aqueous solution of $NH_4Cl$ at 5% by weight, using 10 ml of solution per gram of compound. The suspension was heated at 70°—80°C under reflux condition and with moderate stirring. After 1 hour of heating, the suspension was allowed to settle and the liquid was then rapidly removed. A fresh volume of $NH_4Cl$ solution was added and the suspension was heated again for another hour. The same procedure was repeated several times so that the entire operation lasted 5 hours.

The suspension was filtered and the solid was washed until $Cl^-$ ions were no longer present in the washings. The compound was dried at 120°C for 12 hours and activated in the air at 550°C for 12 hours. The resulting material was the acid form of our composite sample, called HA—48.

Table 5 reports the chemical composition by weight (dried oxide basis), the degree of magnesium leaching (MLD), the Mat/Al ratio, and the degree of crystallinity (DC) of the acid forms of our catalysts: HA stands for composite ZSM—5/asbestos samples under acid forms.

**TABLE 5:** Physical and Chemical Properties of ZSM-5 and Composite Catalysts

| Catalyst[1] | $SiO_2$ (%) | $Al_2O_3$ (%) | $Na_2O$ (%) | MgO (%) | $Fe_2O_3$ (%) | MLD (%) | Mat/Al | DC (%) |
|---|---|---|---|---|---|---|---|---|
| HP-38 | 98.14 | 3.03 | 0.09 | – | – | – | 28.1 | 100 |
| HP-68 | 97.17 | 2.57 | 0.17 | – | – | – | 32.7 | 100 |
| HA-39 | 95.11 | 2.94 | 0.08 | 1.33 | 0.54 | 97.4 | 28.7 | 86 |
| HA-45 | 90.92 | 2.40 | 0.12 | 3.12 | 3.44 | 93.8 | 34.9 | 91 |
| HA-62 | 91.26 | 3.23 | 0.27 | 3.10 | 2.13 | 93.8 | 26.0 | 100 |
| HA-56 | 90.06 | 3.82 | 0.12 | 3.65 | 2.33 | 92.7 | 22.0 | 86 |
| HA-58 | 86.03 | 2.91 | 0.17 | 5.99 | 4.90 | 88.1 | 28.8 | 88 |
| HA-49 | 84.32 | 3.49 | 0.14 | 6.29 | 5.80 | 87.5 | 23.9 | 90 |
| HA-54 | 83.53 | 3.02 | 0.17 | 6.85 | 6.43 | 86.4 | 27.6 | 86 |
| HA-64 | 85.66 | 2.28 | 0.16 | 7.50 | 4.43 | 85.1 | 36.8 | 96 |
| HA-69 | 81.70 | 3.41 | 0.15 | 7.90 | 6.84 | 84.2 | 24.5 | 99 |
| HA-48 | 82.98 | 3.55 | 0.14 | 7.95 | 5.38 | 84.2 | 23.8 | 84 |
| HA-60 | 81.15 | 2.23 | 0.35 | 9.87 | 6.20 | 80.4 | 38.1 | 83 |

(1) Acid form, dried oxide basis.

EP 0 187 594 B1

The magnesium leaching degree (MLD) is defined as:

$$(MLD) = \frac{(MgO)_i - (MgO)_f}{(MgO)_i} \times 100\%$$

where $(MgO)_i$ and $(MgO)_f$ are the magnesium contents (dried oxide basis) of the asbestos fibres and of the catalyst acid forms, respectively.

The (Mat/Al) ratio is:

$$(Mat/Al) = \frac{SiO_2 + Al_2O_3 + Na_2O + MgO + Fe_2O_3}{(Al)}$$

where $SiO_2$, $Al_2O_3$, $Na_2O$, $Fe_2O_3$ are the mole fractions, Al the atom fraction in a dehydrated sample.

In the case of composite catalysts of the present invention, this ratio can be seen as proportional to the reciprocal of the aluminum atom concentration, and thus Brønsted acid site concentration:

$$(Mat/Al) = \frac{constant}{(H^+)}$$

The (Mat/Al) ratio is equivalent to the (Si/Al) ratio commonly used for pure zeolites.

The degree of crystallinity in the pentasil ZSM—5 zeolite of the present composite samples was determined by measuring the area of the diffraction peaks within the range of the Bragg's angle $2\theta = 22.0\text{—}25.0°$. To the pure ZSM—5 zeolite sample (HP—38) was assigned the 100% value of ZSM—5 crystallinity. All measurements were done in the presence of an internal standard, $BaCl_2$, which exhibits a strong diffraction peak at $2\theta = 31.0\text{—}32.5°$. The diffraction powder pattern and the DC measurements were obtained by using a Picker X-ray diffractometer, utilizing the $CuK_\alpha$ ($\lambda = 1.54$ Å) radiation.

Pure ZSM—5 zeolite material

Samples HP—38 and HP—68 were prepared according to a procedure similar to that described in U.S. Patent No. 3,702,886. Table 4 reports the conditions for synthesizing the ZSM—5 samples. The post-synthesis treatments were identical as in the case of the composite material.

Table 5 reports the chemical composition by weight (dried oxide basis), the Mat/Al ratio and the degree of crystallinity (DC) of the acid forms of our catalysts: HP stands for ZSM—5 zeolite samples under acid form.

Zinc loading

Zn ions were incorporated to the H-forms of the zeolite or composite material by contacting the solids with an aqueous solution of $ZnCl_2$ under "ion-exchange conditions".

The following examples can be considered as typical of the zinc loading procedure.

a) HP—68/Zn3 sample:

10 g of HP—68 were brought in contact with an aqueous solution of $ZnCl_2$ (Mallinckrodt) at 2% by weight, using 10 ml of solution per gram of compound. The suspension was heated at 70—80°C under reflux and with moderate stirring. After 3 hours of heating, the suspension was allowed to cool down, then filtered and the solid was washed until $Cl^-$ ions were no longer present in the washings. The compound was dried at 120°C for 12 hours and activated in the air at 550°C for 12 hours.

b) HA—69/Zn4 sample:

9 g of HA—69 were brought in contact with an aqueous solution of $ZnCl_2$ (Mallinckrodt) at 0.5% by weight, using 5 ml of solution per gram of compound. The suspension was moderately stirred at ambient temperature for 15 minutes; then it was filtered and the solid was washed until $Cl^-$ ions were no longer present in the washings. The compound was dried at 120°C for 12 hours and activated in the air at 550°C for 12 hours.

Table 6 reports the preparation conditions and the chemical composition (MgO, $Fe_2O_3$ and Zn contents) of our Zn loaded samples.

| Catalyst | Preparation conditions | | | | | Composition of the Zn loaded catalyst | | | | |
|---|---|---|---|---|---|---|---|---|---|---|
| | W in g of acid-catalyst | ZnCl₂ solution concent. (% w/w) | volume (ml) | Temp. (°C) | Time | Zn (% w/w) | Fe₂O₃ (% w/w) Present | Fe₂O₃ (Δ)(%) | MgO (% w/w) Present | MgO (Δ)(%) |
| HP-68/Zn2 | 10 | 1 | 50 | 70-80 | 0.5 hr | 0.14 | - | - | - | - |
| HP-68/Zn3 | 13 | 2 | 130 | 70-80 | 3 hr | 0.37 | - | - | - | - |
| HP-68/Zn1 | 10 | 2 | 100 | 70-80 | 7 hr | 0.55 | - | - | - | - |
| HA-62/Zn | 22 | 1 | 110 | 70-80 | 0.5 hr | 0.23 | nr | - | nr | - |
| HA-69/Zn4 | 9 | 0.5 | 45 | ambient | 15 mn | 0.32 | 5.82 | -15 | 8.01 | 0 |
| HA-69/Zn1 | 14 | 1 | 70 | ambient | 0.5 hr | 0.53 | 5.38 | -21 | 7.90 | 0 |
| HA-69/Zn2 | 14 | 1 | 70 | ambient | 1 hr | 0.63 | 4.83 | -29 | 7.83 | 0 |
| HA-69/Zn3 | 9 | 1 | 45 | 70-80 | 0.5 hr | 1.25 | 4.59 | -33 | 7.92 | 0 |
| HA-58/Zn2 | 7.9 | 2 | 39.6 | 70-80 | 0.5 hr | 0.88 | nr | - | nr | - |
| HA-58/Zn1 | 10 | 2 | 100 | 70-80 | 1 hr | 1.36 | nr | - | nr | - |

Δ : percentage loss;   nr = not reported

EP 0 187 594 B1

From these data, the following observations must be made:

. with the pure ZSM—5 zeolite sample (HP—68), extremely severe conditions of Zn loading were necessary to obtain a 0.1—0.6% (wt/wt catalyst) metal Zn content. Zn ions were expected to enter into the zeolite structure, mainly to replace part of the $H^+$ acid sites.

. with the composite ZSM—5 zeolite sample (HA—69 and others), very mild conditions of Zn loading were necessary to obtain a 0.1—0.6% (wt/wt catalyst) metal Zn content. Zn content increased with the loading operation while Fe oxide content decreased, the MgO content remained nearly constant. This fact suggests a kind of Fe — Zn exchange at the level of the asbestos remnants.

Preparation of the final catalysts

The acid or the Zn bearing catalysts were intimately mixed with bentonite (20% by weight) and made into pastes with distilled water (1 ml of water per gram of catalyst). The pastes were pressed into 1 mm O.D. extrudates.

Manganese loading

In samples where manganese had to be incorporated, the distilled water which was used in the previous procedure for the final catalyst formation, was replaced by an aqueous solution of Mn with an appropriate concentration of $MnCl_2$, $4H_2O$ from Baker.

Since the amount of the aqueous solution of $MnCl_2$ (in replacement of a corresponding amount of distilled water) was just sufficient to wet the mechanical mixture of "catalyst and bentonite" (technique of dry impregnation), the Mn loading was expected to be effective mostly at the pore openings of the zeolite component.

Table 7 reports the preparation conditions and the Mn content of the final catalysts.

**TABLE 7: Preparation of Zn and Mn loaded catalysts**

| Final Catalyst | "Dry impregnation conditions" | | | | | Mn content |
| | Zn loaded catalyst | | Bentonite | MnCl$_2$ solution | | (metal % w/w) |
| | Zn Content % w/w | W (g) | W (g) | concentr. % w/w H$_2$0 | volume added (ml) | |
|---|---|---|---|---|---|---|
| HP-68/Zn2-Mn2 | 0.14 | 4 | 0.8 | 6 | 3.7 | 1.24 |
| HP-68/Zn3-Mn2 | 0.37 | 4 | 0.8 | 6 | 3.8 | 1.17 |
| HP-68/Zn1-Mn2 | 0.55 | 4 | 0.8 | 6 | 3.7 | 1.72 |
| HA-62/Zn-Mn1 | 0.23 | 4 | 0.8 | 4 | 3.2 | 0.57 |
| HA-62/Zn-Mn2 | 0.23 | 4 | 0.8 | 6 | 3.5 | 1.10 |
| HA-62/Zn-Mn3 | 0.23 | 4 | 0.8 | 10 | 3.6 | 1.79 |
| HA-62/Zn-Mn4 | 0.23 | 4 | 0.8 | 15 | 3.6 | 2.08 |
| HA-69/Zn4-Mn2 | 0.32 | 4 | 0.8 | 6 | 4.0 | 1.60 |
| HA-69/Zn1-Mn2 | 0.53 | 4 | 0.8 | 6 | 4.0 | 1.38 |
| HA-69/Zn1-Mn4 | 0.53 | 4 | 0.8 | 15 | 4.2 | 3.67 |
| HA-58/Zn2-Mn1 | 0.88 | 4.0 | 0.8 | 6 | 3.6 | 1.29 |
| HA-58/Zn1-Mn1 | 1.36 | 4.5 | 0.9 | 6 | 4.4 | 1.15 |

EP 0 187 594 B1

Catalytic experiments

Catalytic tests were performed by injecting methanol from an injection syringe in an infusion pump into a methanol vaporizer and gas mixer. Nitrogen gas was supplied to the methanol vaporizer and gas mixer from a cylinder connected in-line with a flowmeter. The vaporized methanol was then carried in the nitrogen gas through a catalyst bed set in a catalytic reactor which is itself set inside an oven with automatic thermo-regulation. A chromel-alumel thermocouple was placed in the catalyst bed and was used, in conjunction with a digital thermometer unit, to monitor the temperature of the catalyst bed. The gaseous mixture flowing out of the catalytic reactor was run through a series of condensors maintained at 5—10°C, to a liquid collector immersed in an ice bath and a cylinder for gas sampling.

The weight hourly space velocity (WHSV) which is defined as:

$$WHSV = \frac{g \text{ of injected methanol per hour}}{g \text{ of catalyst}}$$

is expressed in $hr^{-1}$.

After a pre-run of 10 minutes, the liquid products were collected and the gaseous ones were analyzed periodically by gas chromatography using a 1.5 m long column packed with the chromosorb P coated with 20% by weight of Squalane. The GC used was a dual FID Hewlett-Packard equipped also with a capillary column (length: 50 m; fused silica coated with a cross-linked polymer) which allowed accurate analyses of the liquid fractions after a run was completed. The composition of the aqueous layer was determined by using a methanol in water external standard.

Table 8 reports the reaction conditions used in the experiments.

## TABLE 8: Reaction conditions

| | |
|---|---|
| Catalyst weight (dehydrated) | 4g |
| Temperature (°C) | 350 – 500 |
| Total Pressure | 1 atm |
| Methanol Pressure | 0.9 atm |
| Inert gas (stripping gas) | nitrogen |
| W.H.S.V. | 2.4 $hr^{-1}$ |
| Reaction duration | 4 hrs |

Table 9 reports the catalytic data of the non-modified catalysts at 400°C with no zinc or manganese. The methanol conversion is the C atom based yield in hydrocarbons. The balance (in C atoms) includes volatile gases ($CO_2$, $H_2$, etc.), dimethyl ether and methanol found in the liquid phase and C deposited onto the catalyst.

It can be expressed as follows:

$$Conversion = \frac{(NC)_{Prod}}{(NC)_{feed}} \times 100$$

where $(NC)_{feed}$ is the number of C atoms in the (methanol) feed and $(NC)_{Prod}$ is the number of C atoms in the (hydrocarbon) products.

The product selectivity is expressed as follows:

$$PS (\%) = \frac{\Sigma_i C_i}{\Sigma C} \times 100$$

where $\Sigma C$ and $\Sigma_i C_i$ are the sums of all hydrocarbons produced and of the selected products, respectively.

TABLE 9: Catalytic data of non-modified catalysts at 400°C

| Catalyst | MLD | Methanol conversion into hydro-carbons(%) | Product selectivities (%) | | | | AR/ liquids (%) | durene/ liquids (%) | AR+OLE (%) | OLE+ liquids (%) | OLE/ AR |
|---|---|---|---|---|---|---|---|---|---|---|---|
| | | | $C_1-C_4$ Para-ffins | $C_2-C_4$ Olefins (OLE) | Aromatics (AR) | Non-Aromatic $C_5^+$ | | | | | |
| HP-38 | – | 99 | 35 | 15 | 26 | 24 | 51 | 1.3 | 41 | 65 | 0.58 |
| HP-68 | – | 99 | 31 | 22 | 26 | 21 | 56 | 1.2 | 48 | 69 | 0.85 |
| HA-39 | 97 | 99 | 37 | 13 | 29 | 21 | 57 | 1.3 | 42 | 63 | 0.45 |
| HA-45 | 94 | 99 | 37 | 15 | 27 | 21 | 57 | 1.3 | 42 | 63 | 0.56 |
| HA-62 | 94 | 99 | 28 | 24 | 25 | 23 | 51 | 1.4 | 49 | 72 | 0.96 |
| HA-56 | 93 | 99 | 34 | 17 | 28 | 21 | 57 | 0.7 | 45 | 66 | 0.61 |
| HA-58 | 88 | 99 | 26 | 26 | 24 | 24 | 50 | 1.1 | 50 | 74 | 1.08 |
| HA-49 | 87 | 99 | 20 | 33 | 20 | 27 | 43 | 1.2 | 53 | 80 | 1.65 |
| HA-54 | 86 | 99 | 17 | 37 | 19 | 27 | 41 | 0.9 | 56 | 83 | 1.95 |
| HA-64 | 85 | 99 | 16 | 39 | 18 | 27 | 39 | 1.2 | 57 | 84 | 2.17 |
| HA-69 | 84 | 99 | 17 | 38 | 18 | 27 | 34 | 1.0 | 56 | 83 | 2.11 |
| HA-48 | 84 | 98 | 16 | 39 | 16 | 29 | 36 | 0.9 | 55 | 84 | 2.44 |
| HA-60 | 80 | 98 | 12 | 44 | 15 | 29 | 34 | 1.2 | 59 | 88 | 2.93 |

EP 0 187 594 B1

Table 10 reports the catalytic data of some of the non-modified catalysts at temperatures higher than 400°C.

TABLE 10: Catalytic data of some non-modified catalysts at 375°C, 450°C and 500°C

| Catalyst | MLD | T (°C) | CH$_3$OH conversion (%) | Product selectivity (%) | | | | Ole+Liq (Liq= Ar+C$_5^+$) | Ole+Ar | Ole/Ar | Durene/ Liq |
| | | | | C$_1$-C$_4$ Paraffins | C$_2$-C$_4$ Olefins (Ole) | Aromatics (Ar) | Non-Aromatics C$_5^+$ | | | | |
|---|---|---|---|---|---|---|---|---|---|---|---|
| HP-38 | – | 375 | 98 | 31 | 17 | 26 | 26 | 69 | 43 | 0.7 | 1.3 |
| HA-39 | 97 | 375 | 99 | 37 | 11 | 28 | 24 | 63 | 39 | 0.4 | 1.4 |
| HA-45 | 94 | 375 | 99 | 37 | 11 | 29 | 23 | 63 | 40 | 0.4 | 1.6 |
| HA-58 | 88 | 375 | 99 | 30 | 18 | 27 | 25 | 70 | 45 | 0.7 | 1.8 |
| HA-48 | 84 | 375 | 97 | 16 | 34 | 16 | 34 | 84 | 50 | 2.1 | 1.4 |
| HA-60 | 80 | 375 | 80 | 9 | 41 | 14 | 36 | 91 | 55 | 2.9 | 2.0 |
| | | | | | | | | | | | |
| HP-38 | – | 450 | 96 | 24 | 38 | 19 | 19 | 76 | 57 | 2.0 | 1.2 |
| HA-45 | 94 | 450 | 99 | 24 | 36 | 22 | 18 | 76 | 58 | 1.6 | 0.9 |
| HA-58 | 88 | 450 | 99 | 17 | 47 | 17 | 19 | 83 | 64 | 2.8 | 0.6 |
| HA-60 | 80 | 450 | 98 | 5 | 63 | 13 | 19 | 95 | 76 | 4.9 | 1.1 |
| | | | | | | | | | | | |
| HA-60 | 80 | 500 | 99 | 4 | 73 | 11 | 12 | 96 | 84 | 6.6 | 0.6 |

EP 0 187 594 B1

Tables 11, 12, 13 and 14 report the catalytic data of all samples in the series HP—68, HA—62, HA—69 and HA—58, respectively.

**TABLE 11: Catalytic data of the pure ZSM-5 based catalysts (series HP-68)**

| Catalyst | Zn (%) | Mn (%) | T (°C) | CH₃OH conversion (%) | (Δ) (%) | C₁-C₄ Paraf. | C₂-C₄ Olefins (Ole) | Aromatics (Ar) | Non-Arom. C₅⁺ | Ole+Liq. (Liq= Ar+C₅⁺) | Ole+Ar | Ole/Ar | Durene/ Liq |
|---|---|---|---|---|---|---|---|---|---|---|---|---|---|
| HP-68 | – | – | 400 | 99 | 0 | 31 | 22 | 26 | 21 | 69 | 48 | 0.9 | 1.2 |
| HP-68/Zn2 | 0.14 | – | 400 | 99 | 0 | 28 | 23 | 27 | 22 | 72 | 50 | 0.9 | 1.6 |
| HP-68/Zn2-Mn2 | 0.14 | 1.24 | 400 | 99 | 0 | 19 | 36 | 22 | 23 | 81 | 58 | 1.6 | 1.5 |
| HP-68/Zn3 | 0.37 | – | 400 | 99 | 0 | 22 | 22 | 36 | 20 | 78 | 58 | 0.6 | 1.7 |
| HP-68/Zn3-Mn2 | 0.37 | 1.17 | 400 | 99 | 0 | 11 | 37 | 31 | 21 | 89 | 68 | 1.2 | 2.4 |
| HP-68/Zn1 | 0.55 | – | 400 | 99 | 0 | 18 | 27 | 35 | 20 | 82 | 62 | 0.8 | 2.4 |
| HP-68/Zn1-Mn2 | 0.55 | 1.72 | 400 | 94 | -5 | 8 | 41 | 25 | 26 | 92 | 66 | 1.6 | 3.5 |
| | | | | | | | | | | | | | |
| HP-68 | – | – | 450 | 99 | 0 | 25 | 32 | 23 | 20 | 75 | 55 | 1.4 | 1.2 |
| HP-68/Zn2 | 0.14 | – | 450 | 99 | 0 | 20 | 36 | 26 | 18 | 80 | 62 | 1.4 | 1.1 |
| HP-68/Zn2-Mn2 | 0.14 | 1.24 | 450 | 97 | -2 | 11 | 48 | 25 | 16 | 89 | 73 | 1.9 | 1.3 |
| HP-68/Zn3 | 0.37 | – | 450 | 97 | -2 | 12 | 36 | 37 | 15 | 88 | 80 | 1.0 | 1.6 |
| HP-68/Zn3-Mn2 | 0.37 | 1.17 | 450 | 93 | -6 | 6 | 46 | 33 | 15 | 94 | 79 | 1.4 | 2.4 |
| HP-68/Zn1 | 0.55 | – | 450 | 91 | -8 | 9 | 38 | 38 | 15 | 91 | 76 | 1.0 | 3.6 |
| HP68/Zn1-Mn2 | 0.55 | 1.72 | 450 | 86 | -13 | 6 | 43 | 35 | 16 | 94 | 78 | 1.2 | 3.8 |

EP 0 187 594 B1

**TABLE 12:** Catalytic data of the composite ZSM-5/asbestos catalysts (series HA-62)

| Catalyst | Zn (%) | Mn (%) | T (°C) | CH₃OH conversion (%) | (A) (%) | C₁-C₄ Paraf. | C₂-C₄ Olefins (Ole) | Aromatics (Ar) | Non-Arom. C₅⁺ | Ole+Liq. (Liq= Ar+C₅⁺) | Ole+Ar | Ole/Ar | Durene/ Liq |
|---|---|---|---|---|---|---|---|---|---|---|---|---|---|
| HA-62 | – | – | 400 | 99 | 0 | 28 | 24 | 24 | 24 | 72 | 48 | 1.0 | 1.4 |
| HA-62/Zn | 0.23 | – | 400 | 99 | 0 | 20 | 27 | 32 | 21 | 80 | 59 | 0.8 | 2.1 |
| HA-62/Zn-Mn1 | 0.23 | 0.57 | 400 | 99 | 0 | 10 | 37 | 34 | 19 | 90 | 71 | 1.1 | 2.0 |
| HA-62/Zn-Mn2 | 0.23 | 1.10 | 400 | 97 | -2 | 10 | 38 | 28 | 24 | 90 | 66 | 1.4 | 2.0 |
| HA-62/Zn-Mn3 | 0.23 | 1.79 | 400 | 97 | -2 | 7 | 42 | 24 | 27 | 93 | 66 | 1.8 | 2.1 |
| HA-62/Zn-Mn4 | 0.23 | 2.08 | 400 | 98 | -1 | 7 | 47 | 21 | 25 | 93 | 68 | 2.2 | 1.7 |
| HA-62 | – | – | 450 | 99 | 0 | 22 | 41 | 20 | 17 | 78 | 61 | 2.1 | 0.8 |
| HA-62/Zn | 0.23 | – | 450 | 99 | 0 | 11 | 40 | 37 | 12 | 89 | 77 | 1.1 | 1.6 |
| HA-62/Zn-Mn1 | 0.23 | 0.57 | 450 | 98 | -1 | 7 | 43 | 39 | 11 | 93 | 82 | 1.1 | 2.7 |
| HA-62/Zn-Mn2 | 0.23 | 1.10 | 450 | 96 | -3 | 6 | 45 | 35 | 14 | 94 | 80 | 1.3 | 2.4 |
| HA-62/Zn-Mn3 | 0.23 | 1.79 | 450 | 98 | -1 | 5 | 52 | 28 | 15 | 95 | 80 | 1.8 | 2.3 |
| HA-62/Zn-Mn4 | 0.23 | 2.08 | 450 | 97 | -2 | 5 | 50 | 32 | 13 | 95 | 82 | 1.6 | 3.1 |

TABLE 13: Catalytic data of the composite ZSM-5/asbestos catalysts (series HA-69)

| Catalyst | Zn (%) | Mn (%) | T (°C) | CH₃OH conversion (%) | (Δ) (%) | C₁-C₄ Paraf. | C₂-C₄ Olefins (Ole) | Aromatics (Ar) | Non-Arom. C₅⁺ | Ole+Liq. (Liq= Ar+C₅⁺) | Ole+Ar | Ole/Ar | Durene/ Liq |
|---|---|---|---|---|---|---|---|---|---|---|---|---|---|
| HA-69 | – | – | 400 | 99 | 0 | 17 | 38 | 18 | 27 | 83 | 56 | 2.1 | 1.0 |
| HA-69/Zn4 | 0.32 | – | 400 | 98 | –1 | 7 | 44 | 27 | 22 | 93 | 71 | 1.6 | 2.4 |
| HA-69/Zn4-Mn2 | 0.32 | 1.60 | 400 | 91 | –8 | 5 | 50 | 18 | 27 | 95 | 68 | 2.8 | 2.2 |
| HA-69/Zn1 | 0.53 | – | 400 | 90 | –9 | 6 | 46 | 25 | 23 | 94 | 71 | 1.8 | 2.7 |
| HA-69/Zn1-Mn2 | 0.53 | 1.38 | 400 | 79 | –20 | 6 | 52 | 16 | 26 | 94 | 68 | 3.3 | 2.8 |
| HA-69/Zn1-Mn4 | 0.53 | 3.67 | 400 | 75 | –24 | 6 | 56 | 11 | 27 | 94 | 67 | 5.1 | 1.9 |
| HA-69 | – | – | 450 | 99 | 0 | 13 | 55 | 15 | 17 | 87 | 70 | 3.7 | 0.8 |
| HA-69/Zn4 | 0.32 | – | 450 | 82 | –17 | 9 | 46 | 31 | 14 | 91 | 77 | 1.5 | 0.4 |
| HA-69/Zn4-Mn2 | 0.32 | 1.60 | 450 | 86 | –13 | 6 | 51 | 25 | 18 | 94 | 76 | 2.0 | 2.7 |
| HA-69/Zn1 | 0.53 | – | 450 | 70 | –29 | 9 | 49 | 31 | 11 | 91 | 80 | 1.6 | 3.7 |
| HA-69/ZN1-Mn2 | 0.53 | 1.38 | 450 | 75 | –24 | 9 | 54 | 23 | 14 | 91 | 77 | 2.3 | 3.5 |
| HA-69/Zn1-Mn4 | 0.53 | 3.63 | 450 | 80 | –19 | 6 | 53 | 24 | 17 | 94 | 77 | 2.2 | 2.7 |

EP 0 187 594 B1

# EP 0 187 594 B1

Discussions on the catalytic performances

By leaching out part of the magnesium (and iron) from the asbestos fibers and then by crystallizing *in situ* the ZSM—5 zeolite within the more or less degraded fibers, we have prepared precursor material for very selective catalysts. In fact, while a high leaching degree (MLD $\geqslant$ 90%) led to a similar product distribution as in pure ZSM—5 zeolite samples, more light olefins were produced mainly at the expenses of light paraffins, with lower MLD (from 75% to 90%) (see Table 9). In particular the medium-leached HA—60 sample (MLD = 80%) exhibits very high selectivity in light olefins at 500°C with no loss in the methanol conversion into hydrocarbons (close to 100%) (see Table 10).

On both ZSM—5 zeolite and composite zeolite-asbestos catalysts, the Zn loading under "ion-exchange" conditions and not exceeding 0.5% (wt/wt) provided a significant increase in liquid (and aromatic) hydrocarbon yield without depleting the methanol conversion (see Tables 11, 12 and 13). When the Zn loading exceeded 0.5 there was a significant loss in the methanol conversion at 400°C (loss of less 20% if the Zn loading ranged between 0.5 and 1.0%). This is shown in Table 14.

TABLE 14: Catalytic data of the composite ZSM-5/asbestos catalysts (series HA-58)

| Catalyst | Zn (%) | Mn (%) | T (°C) | CH$_3$OH conversion | | Product Selectivity (%) | | | | Ole+Liq. (Liq= | Ole+Ar | Ole/Ar | Durene/ |
|---|---|---|---|---|---|---|---|---|---|---|---|---|---|
| | | | | (%) | (Δ) (%) | C$_1$-C$_4$ Paraf. | C$_2$-C$_4$ Olefins (Ole) | Aromatics (Ar) | Non-Arom. C$_5^+$ | Ar+C$_5^+$) | | | Liq |
| HA-58 | – | – | 400 | 99 | 0 | 27 | 26 | 23 | 24 | 73 | 49 | 1.1 | 1.0 |
| HA-58/Zn2 | 0.88 | – | 400 | 86 | –13 | 10 | 32 | 45 | 13 | 90 | 77 | 0.7 | 3.8 |
| HA-58/Zn2-Mn1 | 0.88 | 1.29 | 400 | 88 | –11 | 5 | 38 | 41 | 16 | 95 | 79 | 0.9 | 4.6 |
| HA-58/Zn1 | 1.36 | – | 400 | 94 | –5 | 8 | 35 | 41 | 16 | 92 | 76 | 0.9 | 5.0 |
| HA-58/Zn1-Mn1 | 1.36 | 1.15 | 400 | 79 | –20 | 4 | 46 | 31 | 19 | 96 | 77 | 1.5 | 5.7 |
| | | | | | | | | | | | | | |
| HA-58 | – | – | 450 | 99 | 0 | 17 | 47 | 17 | 19 | 83 | 64 | 2.8 | 5.8 |
| HA-58/Zn2 | 0.88 | – | 450 | 38 | –62 | 15 | 43 | 37 | 5 | 85 | 80 | 1.2 | 6.2 |
| HA-58/Zn2-Mn1 | 0.88 | 1.29 | 450 | 53 | –47 | 7 | 37 | 48 | 8 | 93 | 85 | 0.8 | 4.5 |
| HA-58/Zn1 | 1.36 | – | 450 | 31 | –69 | 10 | 30 | 54 | 6 | 90 | 84 | 0.6 | 5.4 |
| HA-58/Zn1-Mn1 | 1.36 | 1.15 | 450 | 50 | –50 | 10 | 55 | 30 | 5 | 90 | 85 | 1.8 | 5.7 |

EP 0 187 594 B1

The Mn incorporation by "dry impregnation" compressed furthermore the light paraffin formation and varied smoothly the Ole/Ar ratio (depending upon the Mn content of the final catalysts) without practically changing the cumulative production of "Ole + Ar" and "Ole + Liq" (see Tables 11, 12, 13 and 14). This flexibility in the interconversion of light olefins-aromatics is extremely important in terms of industrial application.

## Claims

1. A catalyst selected from ZSM—5 pentasil zeolite catalyst and pentasil zeolite-asbestos composite catalyst wherein the ZSM—5 pentasil zeolite catalyst has a portion of its internal acid reaction sites replaced with zinc ions and has manganese ions adsorbed on the external surface thereof and the pentasil zeolite-asbestos composite catalyst comprises a pentasil zeolite within a magnesium and iron leached asbestos micro-matrix, the pentasil zeolite having a portion of its internal acid reaction sites replaced with zinc ions and manganese ions adsorbed on the external surface of the zeolite while the magnesium and iron leached asbestos micro-matrix is doped internally with zinc ions and externally with manganese ions, the amount of zinc ions in both catalysts being from 0.1 to 1.0% w/w of the metal content and the amount of manganese ion is from 0.2% to 5.0% w/w of the metal content.

2. A catalyst comprising a ZSM—5 pentasil zeolite having a portion of its internal acid reaction sites replaced with zinc ions and manganese ions adsorbed on the external surface, the amount of zinc ions being from 0.1 to 1.0% w/w of the metal content and the amount of manganese ions being from 0.2 to 5.0% w/w of the metal content.

3. The catalyst of Claim 2, wherein the amount of zinc is from 0.1 to 0.6% w/w and the amount of manganese is from 0.2 to 2.5% w/w.

4. A pentasil zeolite-asbestos composite catalyst comprising a magnesium and iron leached asbestos micro-matrix having a pentasil zeolite within its micro-matrix, said pentasil zeolite having a portion of its internal acid reaction sites replaced with zinc ions and having manganese ions adsorbed on the external surface thereof, said magnesium and iron leached asbestos micro-matrix is doped internally with zinc ions and externally with manganese ions, the amount of zinc ions being from 0.1 to 0.5% w/w of the metal content and the amount of manganese being from 0.2 to 5.0% w/w of the metal content.

5. The catalyst of Claim 4, wherein the amount of zinc is from 0.1 to 0.6% w/w and the amount of manganese is from 0.2 to 2.5% w/w.

6. The catalyst of Claim 3, wherein the amount of zinc is 0.37% w/w and the amount of manganese is 1.17% w/w.

7. The catalyst of Claim 5, wherein the amount of zinc is 0.23% w/w and the amount of manganese is 1.10% w/w.

8. The catalyst of Claim 5, wherein the amount of zinc is 0.23% w/w and the amount of manganese is 2.08% w/w.

9. The catalyst of Claim 5, wherein the amount of zinc is 0.32% w/w and the amount of manganese is 1.60% w/w.

10. Process for converting methanol into hydrocarbons having a high cumulative content in light olefins and aromatics, which comprises reacting methanol on a catalyst as claimed in any of claims 1 to 9.

## Patentansprüche

1. Katalysator, der aus dem ZSM—5-Pentasilzeolitkatalysator und dem zusammengesetzten Pentasilzeolit-Asbestkatalysator ausgewählt ist, bei dem in dem ZSM—5-Pentasilzeolitkatalysator ein Teil der internen Säurereaktionsplätze durch Zinkionen ersetzt und Manganionen auf der äußeren Oberfläche adsorbiert sind und der zusammengesetzte Pentasilzeolit-Asbestkatalysator ein Pentasilzeolit innerhalb einer von Magnesium und Eisen ausgelaugten Asbestmikromatrix aufweist, wobei in dem Pentasilzeolit ein Teil der internen Säurereaktionsplätze durch Zinkionen ersetzt und Manganionen auf der äußeren Oberfläche des Zeolit adsorbiert sind, während die von Magnesium und Eisen ausgelaugte Mikromatrix intern mit Zinkionen und extern mit Magnesiumionen dotiert ist, wobei der Betrag der Zinkionen in beiden Katalysatoren von 0,1 bis 1,0% (Massenanteile) des Metallgehaltes und der Betrag der Manganionen von 0,2 bis 5,0% (Massenanteile) des Metallgehaltes beträgt.

2. Katalysator mit einen ZSM—5-Pentasilzeolit, bei dem ein Teil der internen Säurereaktionsplätze durch Zinkionen ersetzt und Magnesiumionen auf der externen Oberfläche adsorbiert sind, wobei der Betrag der Zinkionen von 0,1 bis 1,0% (Massenanteile) des Metallgehaltes und der Betrag der Magnesiumionen von 0,2 bis 5,0% (Massenanteile) des Metallgehaltes beträgt.

3. Katalysator nach Anspruch 2, bei dem der Betrag des Zinks von 0,1 bis 0,6% (Massenanteile) und der Betrag des Magnesiums von 0,2 bis 2.5% (Massenanteile) beträgt.

4. Ein zusammengesetzter Pentasilzeolit-Asbestkatalysator miteiner von Magnesium und Eisen ausgelaugten Asbestmikromatrix mit einem Pentasilzeolit innerhalb der Mikromatrix, wobei bei dem Pentasilzeolit ein Teil der internen Säurereaktionsplätze durch Zinkionen ersetzt und Manganionen auf der äußeren Oberfläche adsorbiert sind, die von Magnesium und Eisen ausgelaugte Asbestmikromatrix intern mit Zinkionen und extern mit Manganionen dotiert ist, der Betrag der Zinkionen von 0,1 bis 0,5% (Massenanteile) des Metallgehaltes und der Betrag des Mangans von 0,2 bis 5,0% (Massenanteile) des Metallgehaltes beträgt.

5. Katalysator nach Anspruch 4, bei dem der Betrag des Zinks von 0,1 bis 0,6% (Massenanteile) und der Betrag des Mangans von 0,2 bis 2,5% (Massenanteile) beträgt.

6. Katalysator nach Anspruch 3, bei dem der Betrag des Zinks 0,37% (Massenanteile) und der Betrag des Mangans 1,17% (Massenanteile) beträgt.

7. Katalysator nach Anspruch 5, bei dem der Betrag von Zink 0,23% (Massenanteile) und der Betrag des Mangans 1,10% (Massenanteile) beträgt.

8. Katalysator nach Anspruch 5, bei dem der Betrag von Zink 0,23% (Massenanteile) und der Betrag des Mangans 2,08% (Massenanteile) beträgt.

9. Katalysator nach Anspruch 5, bei dem der Betrag von Zink 0,32% (Massenanteile) und der Betrag des Mangans 1,60% (Massenanteile) beträgt.

10. Verfahren zum Umwandeln von Methanol in Kohlenwasserstoffe mit einem hohen kumultativen Gehalt an leichten Olefinen und aromatischen Kohlenwasserstoffen, mit Reagieren von Methanol auf einem Katalysator nach einem der Ansprüche 1 bis 9.

**Revendications**

1. Catalyseur choisi entre un catalyseur du type zéolite pentasil ZSM—5 et un catalyseur du type composite zéolite pentasil-amiante, dans lequel le catalyseur du type zéolite pentasil ZSM—5 a une partie de ses sites réactionnels acides internes remplacés par des ions zinc et comporte des ions manganèse adsorbés sur sa surface externe et le catalyseur du type composite zéolite pentasil-amiante contient une zéolite pentasil à l'intérieur d'une micro-matrice d'amiante appauvrie en magnésium et fer par lixiviation, la zéolite pentasil ayant une partie de ses sites réactionnels acides internes remplacés par des ions zinc et comportant des ions manganèse absorbés sur la surface externe de la zéolite tandis que la micro-matrice d'amiante appauvrie en magnésium et fer par lixiviation est dopée intérieurement par des ions zinc et extérieurement par des ions manganèse, la quantité d'ions zinc dans les deux catalyseurs étant de 0,1 à 1,0% en poids/poids du contenu métallique et la quantité d'ions manganèse étant de 0,2% à 5,0% en poids/poids du contenu métallique.

2. Catalyseur comprenant une zéolite pentasil ZSM—5 ayant une partie de ses sites réactionnels acides internes remplacés par des ions zinc et comportant des ions manganèse absorbés sur sa surface externe, la quantité d'ions zinc étant de 0,1 à 1,0% en poids/poids du contenu métallique et la quantité d'ions manganèse étant de 0,2 à 5,0% en poids/poids du contenu métallique.

3. Catalyseur selon la revendication 2, dans lequel la quantité de zinc est de 0,1 à 0,6% en poids/poids et la quantité de manganèse est de 0,2 à 2,5% en poids/poids.

4. Catalyseur du type composite zéolite pentasil-amiante, comprenant une micro-matrice d'amiante appauvrir en magnésium et fer par lixiviation et contenant une zéolite pentasil à l'intérieur de sa micro-matrice, ladite zéolite pentasil ayant une partie de ses sites réactionnels acides internes remplacés par des ions zinc et ayant des ions manganèse adsorbés sur sa surface externe, ladite micro-matrice d'amiante appauvrie en magnésium et fer par lixiviation étant dopée intérieurement par des ions zinc et extérieurement par des ions manganèse, la quantité d'ions zinc étant de 0,1 à 0,5% en poids/poids du contenu métallique et la quantité de manganèse étant de 0,2 à 5,0% en poids/poids du contenu métallique.

5. Catalyseur selon la revendication 4, dans lequel la quantité de zinc est de 0,1 à 0,6% en poids/poids et la quantité de manganèse est de 0,2 à 2,5% en poids/poids.

6. Catalyseur selon la revendication 3, dans lequel la quantité de zinc est de 0,37% en poids/poids et la quantité de manganèse est de 1,17% en poids/poids.

7. Catalyseur selon la revendication 5, dans lequel la quantité de zinc est de 0,23% en poids/poids et la quantité de manganèse est de 1,10% en poids/poids.

8. Catalyseur selon la revendication 5, dans lequel la quantité de zinc est de 0,23% en poids/poids et la quantité de manganèse est de 2,08% en poids/poids.

9. Catalyseur selon la revendication 5, dans lequel la quantité de zinc est de 0,32% en poids/poids et la quantité de manganèse est de 1,60% en poids/poids.

10. Procédé pour convertir du méthanol en hydrocarbures ayant une forte teneur cumulée en oléfines légères et hydrocarbures aromatiques, qui consiste à faire réagir du méthanol sur un catalyseur tel que revendiqué dans l'une quelconque des revendications 1 à 9.